# EUROPÄISCHE PATENTSCHRIFT

(11) **EP 1 245 954 B1**
(45) Veröffentlichungstag und Bekanntmachung des Hinweises auf die Patenterteilung: **24.10.2007**
(21) Anmeldenummer: 02006326.9
(22) Anmeldetag: 21.03.2002
(51) Int. Cl.: G01N 30/16

(54) **Verfahren und Vorrichtung zum Herstellen eines mindestens eine gasförmige Komponente enthaltenden Gasgemisches, insbesondere eines Kalibriergases**
Method and apparatus for generating a gas mixture containing at least one gaseous component, in particular a calibration gas
Procédé et appareil pour préparer des mélanges de gaz contenant au moins un composant gazeux, en particulier un gaz d' étalonnage

(30) Priorität: 27.03.2001 DE 10114947
(43) Veröffentlichungstag der Anmeldung: 02.10.2002
(73) Patentinhaber: Gerstel Systemtechnik GmbH & Co. KG, 45473 Mühlheim an der Ruhr (DE)
(72) Erfinder: Schram, Jürgen, Prof. Dr., 47798 Krefeld (DE); Albinus, Thomas, Dipl.-Ing., 47805 Krefeld (DE)
(74) Vertreter: Sparing Röhl Henseler Patentanwälte European Patent Attorneys

(56) Entgegenhaltungen:
- DE-A- 2 151 007
- GB-A- 2 284 364
- US-A- 4 405 344
- US-A- 4 667 877
- US-A- 5 145 113
- US-A- 5 152 457
- US-A- 5 766 682
- PATENT ABSTRACTS OF JAPAN vol. 011, no. 008 (C-396), 9. Januar 1987 (1987-01-09) & JP 61 186207 A (OLYMPUS OPTICAL CO LTD), 19. August 1986 (1986-08-19)

## Beschreibung

Die Erfindung betrifft ein Verfahren und eine Vorrichtung zum Herstellen eines mindestens eine gasförmige Komponente enthaltenden Gasgemisches, insbesondere eines Kalibriergases, nach dem Oberbegriff des Anspruchs 1 bzw. 8.

Der Einsatz von Gasen mit definierten Konzentrationen an Beimischungen ist sowohl in diversen technischen Einrichtungen als auch als Kalibrierstandards für Analyseverfahren erforderlich.

Bei allen analytischen Einrichtungen stellt die Kalibration den entscheidenden Schritt zum Erhalt richtiger Analysenergebnisse dar. Die apparativen Voraussetzungen einer Analyseeinrichtung können zwar Empfindlichkeit und Präzision eines Analysenergebnisses (und damit die Nachweisgrenze) entscheidend beeinflussen, die Richtigkeit kann aber allein durch Vergleich mit einer Kalibrierprobe bekannten Gehaltes gewährleistet werden.

Im Bereich der Umweltanalytik ist aufgrund der Notwendigkeit einer globalen und zeitunabhängigen Vergleichbarkeit von Analysenergebnissen die Richtigkeit eine entscheidende Größe, um ökologische Prozesse zu verstehen und globale Stoffströme nachvollziehen zu können. Die Analytik gasförmiger Schadstoffe stellt bis in den unteren ppt-Bereich heutzutage für viele Analyseeinrichtungen, wie die Gaschromatographie, kein Problem mehr dar. Weitgehend ungelöst ist jedoch das Problem der adäquaten Kalibration einer solchen Analytik.

Zur Ausblendung eventueller Konditionierungsphänomene ist dabei ein kontinuierlicher Gasstrom des Kalibrationsgases notwendig. Die Anforderungen der Spurenanalytik gasförmiger Schadstoffe machen kontinuierlich strömende zertifizierte Prüfgase aus Druckgasbehältem zum optimalen Kalibrationsmedium. Der hohe Preis, bedingt durch aufwendige Stabilisierung und Herstellung, sowie die produktionsbedingten langen Lieferzeiten, wie auch hohen Mindest-Volumina der einzelnen Behälter lassen diese Verfahren für viele Anwender als zu aufwendig erscheinen. Zudem sind nicht alle Gasmischungen in entsprechenden Drucksystemen und den dort angewandten Metalloberflächen zu stabilisieren. Speziell polare Komponenten sind auf diese Weise nur sehr schwer stabilisiert herstellbar.

Eine ideale Kalibrationsprobe beinhaltet in der Regel eine genau definierte Konzentration des Analyten, möglichst homogen in dem gleichen Matrixgas verteilt, das auch die Analysenprobe umgibt. Eine solche Kalibrationsprobe kann jedoch speziell im Umfeld der Umweltanalytik selten realisiert werden, da es nahezu unmöglich ist, die zur Herstellung der Kalibrationsprobe notwendige unkontaminierte Matrix als Blank und Verdünnungsmedium zu erhalten.

Im Falle der Analytik gasförmiger Proben ergibt sich eine andere Problemlage. Hier ist es zwar relativ einfach, die unkontaminierte Matrix zu erhalten, z.B. synthetische Luft, jedoch stellt sich hier die Herstellung entsprechender Standardproben als problematisch dar.

Gravimetrische Methoden der Gasgemischherstellung sind extrem aufwendig und können nur von Herstellern von Prüfgasen mit großem finanziellen Aufwand hergestellt werden. Hier ergeben sich Probleme aufgrund der im Verhältnis zu den Matrixgasen, aber erst recht zu den Spurenkomponenten großen Massen der das Gasgemisch fassenden Behältnisse.

Bei einer volumetrischen Herstellung müssen kleine Volumina einer oftmals flüssigen Spurenkomponente reproduzierbar in ein sehr großes Volumen des Matrixgases eingebracht werden. Aufgrund der kleinen Dichte der gasförmigen Analyten ergeben sich Probleme mit der Stabilität der Gasgemische. Durch Adsorption und Desorption auf den im Verhältnis zur Masse der Spurenkomponenten sehr großen Oberflächen der mit dem Prüfgas in Kontakt stehenden Gerätschaften besteht das Risiko der Konzentrationsänderung etwa aufgrund von Wanddesorptions- und Wandadsorptionseffekten.

Kommerziell erhältliche Kalibrationssysteme, die dies zu realisieren versuchen, zeichnen sich durch verschiedene Schwachpunkte aus:
Einen kleinen Arbeitsbereich von maximal zwei Dekaden,
Probleme bei niedrigen Konzentrationen,
Beschränkungen bei der Anzahl der zu mischenden Komponenten und deren Verhältnis,
der minimal realisierbare Konzentrationsbereich ist zu hoch.

Es ist auch bekannt, die Reinkomponenten in einen kontinuierlich fließendenden Gasstrom stetig einzubringen und mit diesem reproduzierbar zu homogenisieren. Zur Eintragung kommen hier kritische Düsen, Permeationseinrichtungen oder geregelte Zuströme von höherkonzentrierten Prüfgasen in Frage. Die obigen Probleme werden hierdurch jedoch nicht behoben.

Da die Nachweisgrenze aller analytischen Einrichtungen definitionsgemäß entscheidend von der Reproduzierbarkeit der Einzelergebnisse abhängt, bedeutet jede Verbesserung der Kalibration zudem auch eine Verbesserung der Nachweisgrenze und steigert so erheblich die Leistungsfähigkeit der gesamten Analyseeinrichtung.

Aus DE-A-198 58 366 ist ein Verfahren der eingangs genannten Art bekannt, bei dem ein Kapillardiffusions-Dosiersystem verwendet wird, um ein bestimmtes Mischungsverhältnis eines Trägergases und den interessierenden Komponenten einzustellen. Danach wird das Gemisch über eine Falle geleitet, durch die ein Spülgasstrom geleitet wird, wobei die interessierenden Komponenten in diesen überführt werden. Die dabei erreichbare Genauigkeit ist für manche Anwendungszwecke jedoch keineswegs ausreichend.

Aus US-A-5 400 665 ist ein Verfahren zur Erzeugung eines Analysengasstroms bekannt, bei dem ein Flüssigkeitsnebel aus kleinen Tropfen aus zu einer zu analysierenden Probe erzeugt wird, wobei vor dem Zuführen des Nebels in einen Trägergastrom Lösungsmittel durch Erhitzen des Nebels beseitigt wird. Hierbei werden die Nebeltröpfchen mittels eines Piezoelements erzeugt. Abgesehen davon, daß der Nebel keine im wesentlichen gleichmäßigen Tröpfchen enthält, sondern bei einer Frequenz von 1,3 MHz 70% der Tröpfchen kleiner als 13 µm, wobei die größeren durch Schwerkrafteinwirkung ausgeschieden werden, eignet sich dieses Verfahren nicht zum Herstellen eines mindestens eine gasförmige Spurenkomponente in vorbestimmter Konzentration enthaltenden Gasgemisches, zumal keine Mengendosierung des Probenmaterial vorgenommen wird.

DE2151007 offenbart ein Verfahren und eine Vorrichtung zur Herstellung eines Eich-Dampf-Gas-Gemisches umfassend eine Flasche 1, in der sich eine zu verdampfende Flüssigkeit befindet, ein Rohr 2 durch das ein Inertgas (Stickstoff) eingeleitet wird, eine Kapillare 3, eine Mischkammer 4, in die die Kapillare mündet, und in die von oben Zuluft (10-150 1/min) eingeleitet wird, und eine unterhalb der Mischkammer 4 befindliche Heizpatrone 5. Die Flüssigkeit spritzt aus der Kapillare 3 aus und fällt auf die untere, erhitzte Wand der Mischkammer 4 wo sie verdampft, sich mit der einströmenden Luft vermischt und als Gasgemisch die Mischkammer 4 verläßt.

Aufgabe der Erfindung ist es, ein Verfahren und eine Vorrichtung der eingangs genannten Art zu schaffen, die es ermöglichen, "on-line" eine Gasmischung bekannter Konzentration flüchtiger Komponenten selbst für einen sehr niedrigen Konzentrationsbereich als Kalibrierstandard oder für technische Prozesse bereitzustellen.

Diese Aufgabe wird entsprechend den kennzeichnenden Teilen der Ansprüche 1 und 8 gelöst.

Hierbei wird ein Matrixgasstrom mit einer vorbestimmten Durchflußmenge kontinuierlich gefördert und in den Matrixgasstrom wenigstens eine Kalibrierkomponente in vorbestimmter Menge pro Volumeneinheit Matrixgas eingeführt, wobei die wenigstens eine Kalibrierkomponente in Form von im wesentlichen gleich großen Mikrotröpfchen eingeführt und in dem Matrixgas zur Bildung des Kalibriergases oder Muttergemisches verdampft wird, wobei die Mikrotröpfchen von Düsen abgegeben werden, die jeweils über ein durch entsprechend der vorgesehenen Abgabemenge erzeugten Auslöseimpulse getriggertes Piezoelement kontraktiv beaufschlagt werden. Hierdurch ergibt sich ein kontinuierlicher Kalibriergasstrom mit einer bekannten Konzentration flüchtiger, insbesondere leichtflüchtiger Komponenten insbesondere unter Normaldruck. Dabei können verdünnte Gasmischungen verschiedenster Komponenten in den unterschiedlichsten Konzentrationsverhältnissen "on-line" erzeugt und dem Probeneintragssystem einer zu kalibrierenden gasanalytischen Einrichtung zugänglich gemacht werden. Gegebenenfalls kann die Kalibrierkomponente gekühlt werden, um im flüssigen Zustand vorzuliegen und als Mikrotröpfchen eingebracht werden zu können.

Zur Erzeugung von Vielkomponenten-Gasmischungen kann entweder eine Lösung, die die Komponenten enthält, mit einem Dosierkopf oder es können entsprechend der Anzahl an Komponenten mehrere Dosierköpfe verwendet werden.

Auf diese Weise ist es möglich, einen hochgenau geregelten Probeneintrag in einen kontinuierlichen Gasstrom zu gewährleisten und somit ideale Voraussetzungen für eine flexibles Kalibrationssystem zu bieten. In einen über einen exakten Massendurchflußregler (Mass-Flow-Controler) geregelten Trägergasstrom (insbesondere N 2 oder synthetische Luft) werden aus einem oder vorzugsweise aus mehreren, jeweils verschiedene Komponenten fördernden Mikrotröpfchen-Abgabeeinrichtungen die zur Kalibration erforderlichen Stoffmengen an Analyt eingebracht. Durch separates Ansteuern jeder Mikrotröpfchen-Abgabeeinrichtung können die verschiedensten Mischungsverhältnisse der Komponenten in der Gasphase realisiert werden. Aufgrund der zu erreichenden geringen Tröpfchengröße (im Bereich von beispielsweise etwa 20 bis 100 µm, insbesondere etwa 30 bis 50 µm) wird zudem ein schnelles vollständiges Verdampfen der Komponenten in dem Trägergasstrom gewährleistet werden. Dies hat ein homogenes Kalibrier- oder Prüfgas zur Folge. Die Möglichkeit 1 bis 2000 Tropfen/sec eines Volumens etwa von 15 bis 65 pl jeder Komponente in einen gegebenen Gasstrom einzubringen, ermöglicht hier allein eine Kalibration über mehr als vier Konzentrations-Dekaden, ohne die Einrichtung mechanisch verändern zu müssen. Somit weist ein solches Verfahren eine deutlich größere Flexibilität bezüglich Konzentrationsbereich und Komponentenverhältnissen auf, als die kommerziell bisher erhältlichen Verfahren bzw. Prüfgase.

So läßt sich die Richtigkeit der Analyseergebnisse definierter Standardproben als auch realer Proben gegenüber üblichen Kalibrationstechniken bei geringen Kosten deutlich zu steigern und somit auch die Reproduzierbarkeit und in Folge dessen zusätzlich die Nachweisgrenze deutlich senken. Ferner steht das Kalibriergas für einen schnellen Einsatz etwa im Laboratorium bereit.

Nach Bestimmung der Tröpfchengröße - gravimetrisch oder mittels Bestimmung der Strömungsgeschwindigkeit in einer Kapillarstrecke - kann die Kalibriererzeugung digital und somit leicht automatisierbar - durch digitale Einstellung der Tropfenfrequenz - auf die gewünschte Prüfgaskonzentration eingestellt werden. Zudem kann diese Konzentration während des Ablaufes stetig digital geändert werden und so eine automatische Kalibrationsreihe an verschiedenen Prüfgaskonzentrationen generiert werden. Ein weiterer Vorteil ist darin zu sehen, das es gelingt, auch Prüfgase polarer Komponenten zu generieren, die in handelsüblichen Druckgasbehältem nicht erhältlich sind.

Die gleichmäßige Größe und Abfolge der Tropfen gewährleistet dabei die Bildung eines homogenen Gasgemisches, wie es zur Kalibration optimal ist. Eine digitale Steuerung der Mikrotröpfchen-Dosiereinrichtung erlaubt es, pro Sekunde 1 bis 2000 Tropfen eines Volumens von 15 pl (bei einem Düsendurchmesser von d=30 µm) jeder Komponente in einen gegebenen Gasstrom einzubringen. Somit wird eine Kalibration über mehr als vier Dekaden möglich, ohne das System mechanisch zu verändern. Um ein optimal kalibriertes Gasgemisch zu erhalten, ist die Größe und Homogenität der einzelnen gebildeten Tropfen von entscheidender Bedeutung. Bei optimaler Größe der Tröpfchen hängt es allein von der Homogenität der Größenverteilung ab, wie groß im Endeffekt das Flüssigkeitsvolumen ist, mit dem sich die Gesamtmenge der Spurenkomponenten im Matrixgas verteilt. Die Erzeugung der Mikrotröpfchen zeichnet sich durch eine extrem reproduzierbare Arbeitweise aus, die Streuung der einzelnen Tropfengrößen liegt, auf die Masse bezogen, bei etwa 1% und somit deutlich unter den Streuungen vergleichbarer Verfahren. Der Umstand, daß die Kalibrierkomponenten als Mikrotröpfchen und nicht etwa als Flüssigkeitsfilm in das Matrixgas eingetragen wird, führt dazu, daß der Verdampfungssprozess bei gleicher Tropfengröße sehr reproduzierbar abläuft und so ein homogenes Gasgemisch gebildet werden kann.

Der notwendige exakte Volumenstrom des Matrixgases in der Kalibrationseinheit kann durch Mass-Flow-Controler sichergestellt werden. Dieses Regelinstrument wird zweckmäßigerweise auf der Matrixgasseite eingesetzt, um es ideal kalibrieren zu können und eventuelle störende Einflüsse der oftmals reaktiven und damit korrosiven Spurenkomponente auszuschließen.

Die durch das Verdampfen der Spurenkomponenten zu erwartenden Volumenvergrößerungen können rechnerisch erfaßt werden und bei der Steuerung der Mikrotröpfchen-Dosiereinrichtungen berücksichtigt werden.

Das Verfahren ist universell für alle Methoden der Analytik leichtflüchtiger Komponenten (beispielsweise VOC's und WOC's) einsetzbar. Diese gasanalytischen Methoden spielen eine immer stärkere Rolle in den stetig wichtiger werdenden Anwendungsgebieten der instrumentellen Analytik, wie z.B. der Umweltanalytik (Klimaschutz) oder der Analytik im Umfeld von Arbeits- und Gesundheitsschutz. Erfingungsgemäß hergestellte Kalbriergase lassen sich in gaschromatographischen Einrichtungen, Gasphasenadsorptions- und - desorptions-einrichtungen, in der Infrarotspektroskopie, in der UVNIS-Spektroskopie oder in Gasphasen-Überwachungseinrichtungen verwenden.

Neben Anwendungen in der chemischen Analytik sind weitere Arbeitsfelder auch Bereiche, in denen idealerweise definierte Gasphasen einsetztbar sind, wie Klimakammern, Sensor-Prüfstände oder Gasphasen-Epitaxie-Systeme. Das Verfah-ren kann auch dazu verwendet werden, um beispielsweise bestimmte Gase wie etwa Erdgas oder Atmosphären, etwa Luft, mit einem definierten Duftstoffgehalt und/oder zu versehen.

Die quantitative Bestimmung der verschiedenen gasförmigen Schadstoffe in Atmosphäre und Umgebungsluft hat große Bedeutung sowohl für den Klimaschutz, als auch für den Arbeits- und Gesundheitsschutz. Speziell in einer Industriegesellschaft sind die ökologischen Wirkungen von gasförmigen Schadstoffen aufgrund ihrer hohen Diffusionsraten und ihrer weitreichenden Transportmöglichkeiten in dem Umweltkompartiment Luft für den Eintrag in die Ökosphäre von großer Bedeutung. Kontaminationen des Umweltkompartiments Luft durch die verschiedensten gasförmigen Schadstoffe spielen somit gerade in Bezug auf direkte Schädigungen, aber auch Anreicherungen in Ökosystemen eine wichtige Rolle. Von besonderem Interesse sind dabei richtige Analysenergebnisse gerade einzelner Spurenkomponenten, da diese oftmals aufgrund ihrer ökotoxikologischen Wirkung sehr schädliche Einflüsse auf Ökosysteme ausüben. Exemplarisch seien hier nur die schädigende Wirkung leichtflüchtiger Kohlenwasserstoffe (VVOC und VOC) erwähnt. Die Richtigkeit der Analytik dieser Stoffe, die sich oftmals aufgrund einer Persistenz in Ökosystemen anzureichern vermögen, muß ständig verbessert werden. Nur so können alle Eintragspfade, auch die natürlichen, gering konzentrierten global und auch über längere Zeiträume hinweg, richtig erfasst werden.

Weitere Ausgestaltungen der Erfindung sind der nachfolgenden Beschreibung und den Unteransprüchen zu entnehmen.

Die Erfindung wird nachstehend anhand von beigefügten Abbildungen näher erläutert.

Fig. 1 zeigt schematisch eine Ausführungsform einer Einrichtung zur Erzeugung eines Kalibriergases.

Fig. 2 zeigt schematisch eine Mikrotröpfchen-Bildung durch eine Düse einer Mikrotröpfchen-Dosiereinrichtung.

Fig. 3 zeigt schematisch eine Thermodesorptionseinrichtung zum Probeneintrag für einen Gaschromatographen.

Fig. 4 zeigt schematisch eine weitere Mikrotröpfchen-Dosiereinrichtung für eine Thermodesorptionseinrichtung eines Gaschromatographen.

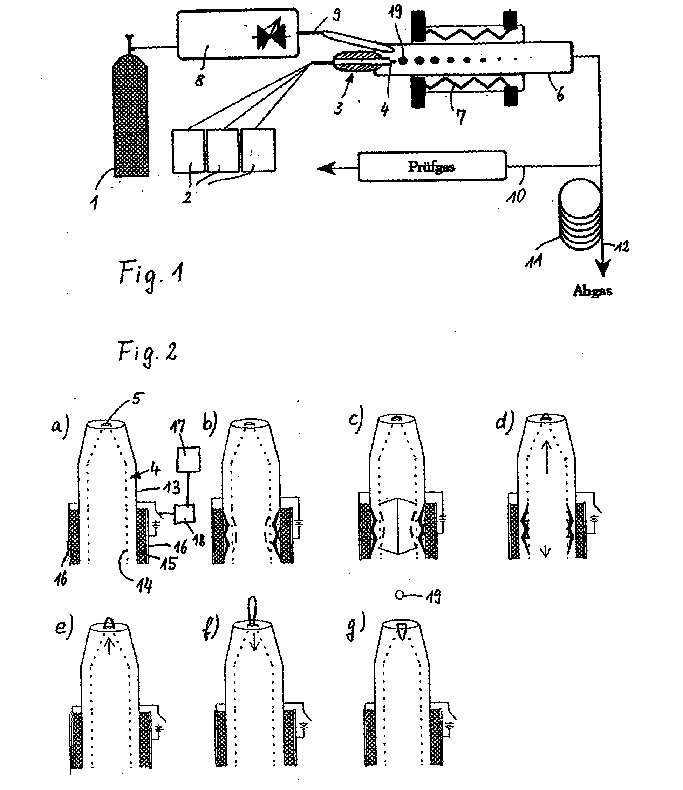

Gemäß Fig. 1 sind eine Matrixgasquelle 1, etwa eine Reinstgas enthaltende Gasflasche, und Quellen 2 für verschiedene Kalibrierkomponenten, die flüssig vorliegen, vorgesehen. Letztere sind an eine Mikrotröpfchen-Dosiereinrichtung 3 angeschlossen, die eine entsprechende Anzahl von Düsen 4 umfaßt, deren Austrittsöffnungen 5 (sh. Fig. 2) in den Innenraum eines Verdampferrohrs 6 gerichtet sind. Das Verdampferrohr 6 ist von einer Heizeinrichtung 7, etwa einer Heizwendel aus einem Widerstandsdraht, umgeben und eintrittsseitig, d.h. benachbart zur Mikrotröpfchen-Dosiereinrichtung 3 an eine über einen Mass-Flow-Controler 8 geregelte Leitung 9 angeschlossen. Das Verdampferrohr 6 ist gesteuert auf eine vorbestimmte Temperatur durch die Heizeinrichtung 7 heizbar. An der Austrittsseite ist das Verdampferrohr 6 an eine Abführleitung 10 für Kalibriergas angeschlossen. Von dieser zweigt außerdem eine eine als Rückdiffusionsschutz dienende Kapillare 11 aufweisende Abgasleitung 12 für zuviel erzeugtes Kalibriergas ab.

Jede Düse 4 umfaßt, wie in Fig. 2a schematisiert dargestellt, einen Düsenkörper 13 mit einer mit der zugehörigen Quelle 2 verbundenen Innenbohrung 14, die zu einer verengten Austrittsöffnung 5 führt. Der Düsenkörper 13 ist von einem Piezoelement 15 umgeben, dessen an der Außenseite befindliche Elektroden 16 an einen von einer insbesondere digitalen Steuerung 17 triggerbaren Dosierimpulsgeber 18 angeschlossen sind. Durch Anlegen eines Spannungsimpulses als Dosierimpuls verformt sich das Piezoelement 15 und damit die Wandung des Düsenkörpers 13, die hierdurch kontrahiert, vgl. Fig. 2b. Hierdurch läuft eine Druckwelle in beiden Richtung der Längsachse der Düse 4, Fig. 2c. Hierdurch wird die Flüssigkeit in der Innenbohrung 14 entsprechend beschleunigt und eine bestimmte Menge an Flüssigkeit tritt aus der Austrittsöffnung 5 heraus. Am Ende des Spannungsimpulses findet eine Druckabnahme statt, da die Kontraktion zurückgeführt wird. Eine Unterdruckwelle läuft in beiden Richtungen, Fig. 2d. Die Unterdruckwelle erreicht die Austrittsöffnung 5, die Flüssigkeit wird abgebremst, Fig. 2e. Die Flüssigkeit schwingt zurück und ein Mikrotröpfchen 19 schnürt sich ab, Fig. 2f. Ein Nachschwingen der Flüssigkeitssäule einwärts findet statt, Fig. 2g.

Die gepulste Kontraktion des Düsenkörpers 13 führt zu einer entsprechenden pulsartigen Abgabe von Mikrotröpfchen 19, die ein feinteiliges Aerosol bilden und aufgrund ihrer geringen Größe und großen Oberfläche in dem Verdampferrohr 6 vollständig verdampfen.

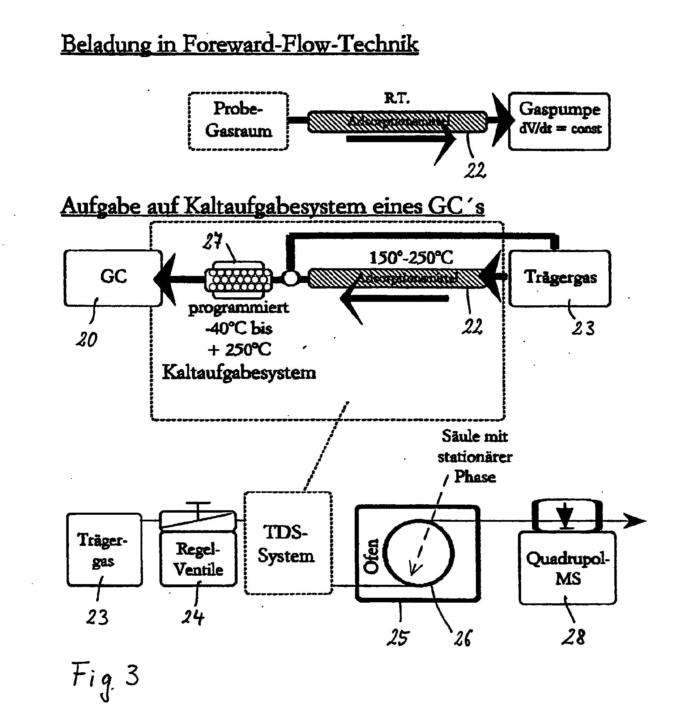

Sehr gut mit einem Gaschromatographen 20 koppelbar hat sich eine Thermodesorptionseinrichtung 21 als Probenahme / Probeneintragssystem (TDS) erwiesen. Hierbei wird, wie in Fig. 3 dargestellt, zur Probenahme ein großes Gasvolumen von einigen Litern bis zu einigen m³ durch ein mit einem Adsorptionsmedium gefülltes Röhrchen 22 gesaugt und dabei die Spurenkomponenten auf der Füllung (z.B. Silikagel oder Tenax) adsorbiert und so angereichert. Zur Analyse wird das Röhrchen 22 dann programmiert aufgeheizt, dabei von einem von einer Trägergasquelle 23 über Regelventile 24 gesteuert kommenden Trägergas durchströmt, das die unter diesen Bedingungen desorbierenden Spurenkomponenten in den Gaschromatographen 20 einträgt. Dieser Eintrag kann entweder direkt auf eine in einem Ofen 25 angeordnete Trennsäule 26 erfolgen, oder aber zunächst zur Anreicherung in eine ebenfalls programmierbar aufheizbare Kühlfalle 27 (z.B. Kaltaufgabesystem). Speziell bei dieser Art des Probeneintrags stellt sich das Problem der Kalibration. Es ist nämlich nicht davon auszugehen, daß der Adsorptionsgrad und somit dann die Wiederfindungsrate gerade für Spurenkomponenten 100 % beträgt. Aus diesem Grunde ist es hier, abweichend von der zur Zeit geübten Praxis, notwendig, das System mit einem kontinuierlichen Prüfgasstrom zu kalibrieren. Die Trennsäule 26 ist hierbei an ein Analysegerät 28, etwa einen Quadrupol-Massenspektrographen angeschlossen.

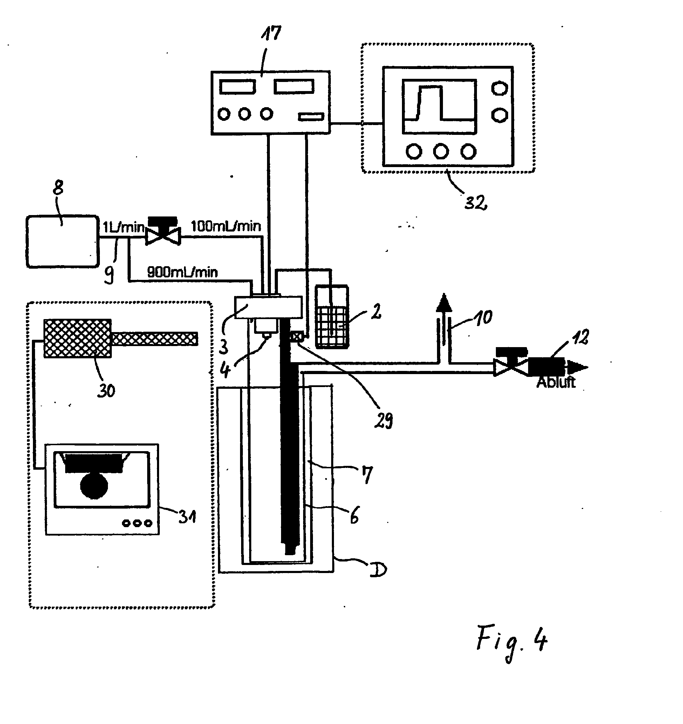

Wie in Fig. 4 dargestellt, kann der dort dargestellten Ausführungsform der Mikrotropfen-Dosiereinrichtung 3 Matrixgas, etwa Reinstgas wie Stickstoff, über den Mass-Flow-Controler 8 gesteuert zur Kalibriergaserzeugung in zwei unterschiedlichen Massenströmen hier von 900 ml/min und über ein geöffnetes Ventil 100 ml/min zugeführt werden. Der Matrixgasstrom kann unter Reinstgasbedingungen durch die Mikrotröpfchen-Dosiereinrichtung 3 mit einer oder mehreren gaschromatographisch empfindlich bestimmbaren Komponenten aus Quellen 2 versetzt werden. Hierbei dient der geringere Massenstrom von 100 ml/min zum Umspülen der Düsen 4, um die Mikrotröpfchen 19 in das Verdampferrohr 6 weiterzutragen. Das Verdampferrohr 6 ist hier U-förmig zur Abführleitung 10 geführt und insgesamt von der Heizeinrichtung 7 umgeben. Das Verdampferrohr 6 und die Heizeinrichtung 7 sind in einem Dewargefäß D zur Vergleichmäßigung der Wärmeverteilung in dem Verdampferrohr 6 angeordnet.

Hierbei ist ferner eine optische Kontrolle der Mikrotröpfchenbildung vorgesehen, die eine Stroboskopdiode 29 umfaßt, die mittels einer Kamera 30 beobachtet wird, die ihrerseits an einen Monitor 31, mit dem die Dosierung visuell kontrolliert werden kann angeschlossen ist. Außerdem ist an die Steuerung S ein Oszilloskop 32 angeschlossen, das zur Kontrolle des Dosierimpulses dient. Die Abführleitung 10 ist hier mit einem Ventil versehen.

Wenn zur Erzeugung von Vielkomponenten-Gasmischungen eine Lösung, die die Komponenten enthält, mit nur einem Dosierkopf und damit nur einer Düse dosiert wird, kann diese beispielsweise durch Verwendung einer methanolischen Lösung mit z.B. max. 1 Gew.-% Fremdanteil, d.h. Kalibrierkomponenten oder Analyten, angemischt werden. Das bedeutet, daß die für den Betrieb entscheidenden Parameter der Lösung, also Viskosität und Benetzungsverhalten, gleich bleiben, da sie hauptsächlich vom Methanol abhängen. Damit können bei gleichbleibenden Geräteparametern verschiedenste Gasmischungen hergestellt werden. Allerdings ist hierbei auch immer das Lösungsmittel, etwa Methanol, im Gas enthalten. Dieser Lösungsmittelanteil ist aber gegenüber bekannten Verfahren, die mit Zudosierungen von Lösungen arbeiten, sehr gering.

Die mechanischen Bauteile der Vorrichtung sind unabhängig von der herzustellenden Mischung, eine Veränderung der Konzentration der Mischung wird nur durch Veränderung einer digital einzustellenden Größe, nämlich der Frequenz der Tropfenabgabe, also ohne Änderung von Volumenströmen von Gasen erzielt. Dies ist mit sehr hoher Genauigkeit reproduzierbar und entsprechend zuverlässig.

## Patentansprüche

1. Verfahren zum Herstellen eines mindestens eine gasförmige Spurenkomponente in vorbestimmter Konzentration enthaltenden Gasgemisches, insbesondere eines Kalibriergases, wobei ein Matrixgasstrom mit einer vorbestimmten Durchflußmenge kontinuierlich gefördert und in den Matrixgasstrom wenigstens eine Spurenkomponente in vorbestimmter Menge pro Volumeneinheit Matrixgas eingeführt wird, wobei die wenigstens eine Spurenkomponente in Form von aufeinanderfolgenden, im wesentlichen gleich großen Mikrotröpfchen eingeführt und in dem Matrixgas zur Bildung des Gasgemisches verdampft wird, wobei die Mikrotröpfchen von mindestens einer Düse (4) abgegeben werden, die jeweils über ein durch entsprechend einer vorgesehenen Abgabemenge pro Zeiteinheit erzeugten Dosierimpulse getriggertes Piezoelement (16) kontraktiv beaufschlagt wird.

2. Verfahren nach Anspruch 1, **dadurch gekennzeichnet, daß** der die Mikrotröpfchen enthaltende Matrixgasstrom beheizt wird.

3. Verfahren nach Anspruch 1 oder 2, **dadurch gekennzeichnet, daß** die durch die Verdampfung und gegebenenfalls durch Beheizung bewirkte Volumenexpansion bei der Steuerung der Durchflußmenge des Matrixgasstroms berücksichtigt wird.

4. Verfahren nach einem der Ansprüche 1 bis 3, **dadurch gekennzeichnet, daß** als Matrixgas ein Reinstgas, insbesondere synthetische Luft oder Stickstoff, verwendet wird.

5. Verfahren nach einem der Ansprüche 1 bis 4, **dadurch gekennzeichnet, daß** Mikrotröpfchen einheitlicher Größe des Durchmessers aus dem Bereich von etwa 30 bis 50 µm eingeführt werden.

6. Verfahren nach einem der Ansprüche 1 bis 5, **dadurch gekennzeichnet, daß** die Mikrotröpfchen durch einen sanften, die Düsen (4) umspülenden Matrixgasteilstrom von den Düsen (4) weggetragen werden.

7. Verfahren nach einem der Ansprüche 1 bis 6, **dadurch gekennzeichnet, daß** eine oder mehrere Komponenten in Form einer diese in vorbestimmter Konzentration enthaltenden Lösung tropfenweise eingeführt werden.

8. Vorrichtung zum Herstellen eines mindestens eine gasförmigen Spurenkomponente in vorbestimmter Konzentration enthaltenden Gasgemisches, insbesondere eines Kalibriergases, mit einer Matrixgasquelle (1), der ein Massenstromregler nachgeschaltet ist, und wenigstens einer Quelle (2) für eine Komponente sowie mit einer Dosiereinrichtung (3) für die wenigstens eine Spurenkomponente, wobei die Dosiereinrichtung (3) eine Mikrotröpfchen-Dosiereinrichtung mit wenigstens einer einzelne Mikrotröpfchen aufeinanderfolgend abgebende Düse (4) ist, die in ein von dem Matrixgas durchströmtes Verdampferrohr (6) mündet und zwecks Erzeugung von Mikrotröpfchen als Mittel zum kontraktiven Beaufschlagen hiervon ein Piezoelement (16) aufweist, das mit einer Steuerung (17) gekoppelt ist, die das Piezoelement (16) mit Dosierimpulsen entsprechend der vorgesehenen Abgabemenge pro Zeiteinheit triggert.

9. Vorrichtung nach Anspruch 8, **dadurch gekennzeichnet, daß** das Verdampferrohr (6) beheizbar ist.

10. Vorrichtung nach Anspruch 8 oder 9, **dadurch gekennzeichnet, daß** für die Mikrotröpfchen-Dosiereinrichtung (3) eine digitale Steuerung (S) für die Länge und Folge der Dosierimpulse vorgesehen ist.

11. Vorrichtung nach einem der Ansprüche 8 bis 10, **dadurch gekennzeichnet, daß** die Düsen (4) von einem in das Verdampferrohr (6) gerichteten, sanften Matrixgasteilstrom umspülbar sind.

12. Verfahren zum Kalibrieren einer gasanalytischen Einrichtung, insbesondere eines Gaschromatographen (20), durch Hindurchführen und Analysieren eines gasförmigen, mindestens eine Kalibrierkomponente enthaltenden Matrixgases, wobei ein Matrixgasstrom mit einer vorbestimmten Durchflußmenge in die Einrichtung eingeleitet, in den Matrixgasstrom vor dessen Hindurchführen durch die Einrichtung wenigstens eine Kalibrierkomponente in vorbestimmter Menge pro Volumeneinheit Matrixgas in Form von im wesentlichen gleich großen Mikrotröpfchen eingeführt und in dem Matrixgas zur Bildung eines Kalibriergases verdampft wird, wobei die Mikrotröpfchen von wenigstens einer Düse (4) abgegeben werden, die jeweils über ein durch entsprechend der vorgesehenen Abgabemenge pro Zeiteinheit erzeugten Dosierimpulse getriggertes Piezoelement (16) kontraktiv beaufschlagt wird.

13. Verfahren nach Anspruch 12, **dadurch gekennzeichnet, daß** der Matrixgasstrom mit den Mikrotröpfchen beheizt wird.

14. Verfahren nach Anspruch 12 oder 13, **dadurch gekennzeichnet, daß** die durch die Verdampfung und gegebenenfalls durch Beheizung bewirkte Volumenexpansion bei der Steuerung der Durchflußmenge des Matrixgasstroms berücksichtigt wird.

15. Verfahren nach einem der Ansprüche 12 bis 14, **dadurch gekennzeichnet, daß** als Matrixgas ein Reinstgas, insbesondere synthetische Luft oder Stickstoff, verwendet wird.

16. Verfahren nach einem der Ansprüche 12 bis 15, **dadurch gekennzeichnet, daß** Mikrotröpfchen einheitlicher Größe des Durchmessers im Bereich von etwa 30 bis 50 µm eingeführt werden.

17. Verfahren nach einem der Ansprüche 12 bis 16, **dadurch gekennzeichnet, daß** die Mikrotröpfchen durch einen sanften, die Düsen (4) umspülenden Matrixgasteilstrom von den Düsen (4) weggetragen werden.

18. Verfahren nach einem der Ansprüche 12 bis 17, **dadurch gekennzeichnet, daß** eine oder mehrere Kalibrierkomponenten in Form einer diese in vorbestimmter Konzentration enthaltenden Lösung tropfenweise eingeführt werden.

19. Verfahren nach einem der Ansprüche 1 bis 7 oder 12 bis 18, **dadurch gekennzeichnet, daß** die Konzentration der Spurenkomponente(n) durch Verändern der Frequenz der Triggerimpulse verändert wird.

## Claims

1. Process for producing a gas mixture which contains at least one gaseous trace component in a predetermined concentration, in particular for producing a calibration gas, in which a matrix gas stream is conveyed continuously with a predetermined quantitative flow, and at least one trace component is introduced into the matrix gas stream in a predetermined quantity per unit volume of matrix gas, wherein the at least one trace component is introduced in the form of successive microdroplets of substantially the same size and is evaporated in the matrix gas to form the gas mixture, the microdroplets being delivered from at least one nozzle (4), which is in each case made to contract by means of a piezoelement (16) which is triggered by metering pulses which are generated according to an intended delivery quantity per unit time.

2. Process according to Claim 1, **characterized in that** the matrix gas stream which contains the microdroplets is heated.

3. Process according to Claim 1 or 2, **characterized in that** the volumetric expansion effected by the evaporation and, if appropriate, by heating is taken into account during the control of the quantitative flow of the matrix gas stream.

4. Process according to one of Claims 1 to 3, **characterized in that** the matrix gas used is an ultra pure gas, in particular synthetic air or nitrogen.

5. Process according to one of Claims 1 to 4, **characterized in that** microdroplets with a uniform diameter from the range from approximately 30 to 50 µm are introduced.

6. Process according to one of Claims 1 to 5, **characterized in that** the microdroplets are carried away from the nozzles (4) by a gentle matrix gas part stream which flushes around the nozzles (4).

7. Process according to one of Claims 1 to 6, **characterized in that** one or more components are introduced dropwise in the form of a solution containing these components in a predetermined concentration.

8. Device for producing a gas mixture which contains at least one gaseous trace component in a predetermined concentration, in particular for producing a calibration gas, comprising a matrix gas source (1), downstream of which there is a mass flow controller, and at least one source (2) for a component, and a metering device (3) for the at least one trace component, wherein the metering device (3) is a microdroplet-metering device with at least one nozzle (4) which releases successive individual microdroplets, opens out into an evaporator tube (6), through which the matrix gas flows, and, to produce microdroplets, comprises a piezoelement (16) as a means to make the nozzle 4 contract, which piezoelement (16) is coupled to a control unit (17) which triggers the piezoelement (16) with metering pulses according to the intended delivery quantity per unit time.

9. Device according to Claim 8, **characterized in that** the evaporator tube (6) is heatable.

10. Device according to Claim 8 or 9, **characterized in that** a digital control unit (S) for controlling the length and sequence of the metering pulses is provided for the microdroplet-metering device (3).

11. Device according to one of Claims 8 to 10, **characterized in that** a gentle matrix gas part-stream, which is directed into the evaporator tube (6), can flush around the nozzles (4).

12. Process for calibrating a gas analysis device, in particular a gas chromatograph (20), by passing through and analysing a gaseous matrix gas which contains at least one calibration component, wherein a matrix gas stream is introduced into the device at a predetermined quantitative flow, at least one calibration component, before it is passed through the device, is introduced into the matrix gas stream in a predetermined quantity per unit volume of matrix gas, in the form of microdroplets of substantially identical size and is evaporated in the matrix gas to form a calibration gas, the microdroplets being delivered by at least one nozzle (4), which is in each case made to contract by means of a piezoelement (16) which is triggered by metering pulses which are generated according to the intended delivery quantity per unit time.

13. Process according to Claim 12, **characterized in that** the matrix gas stream together with the microdroplets is heated.

14. Process according to Claim 12 or 13, **characterized in that** the volumetric expansion which is effected by the evaporation and, if appropriate, by heating is taken into account during the controlling of the quantitative flow of the matrix gas stream.

15. Process according to one of Claims 12 to 14, **characterized in that** the matrix gas used is an ultra pure gas, in particular synthetic air or nitrogen.

16. Process according to one of Claims 12 to 15, **characterized in that** microdroplets with a uniform diameter in the range from approximately 30 to 50 µm are introduced.

17. Process according to one of Claims 12 to 16, **characterized in that** the microdroplets are carried away from the nozzles (4) by a gentle matrix gas part stream which flushes around the nozzles (4).

18. Process according to one of Claims 12 to 17, **characterized in that** one or more calibration components are introduced dropwise in the form of a solution containing these components in a predetermined concentration.

19. Process according to one of Claims 1 to 7 or 12 to 18, **characterized in that** the concentration of the trace component(s) is changed by changing the frequency of the trigger pulses.

## Revendications

1. Procédé pour préparer un mélange gazeux contenant au moins une composante gazeuse de traçage en concentration prédéterminée, en particulier un gaz de calibrage, dans lequel on alimente en continu un courant gazeux matriciel avec un débit quantitatif prédéterminé et l'on introduit dans le courant gazeux matriciel au moins une composante de traçage en quantité prédéterminée par unité volumétrique du gaz matriciel, dans lequel ladite au moins une composante de traçage est introduite sous la forme de microgouttelettes successives essentiellement de même taille et est vaporisée dans le gaz matriciel pour former le mélange gazeux, les microgouttelettes sont distribuées par au moins une buse (4) qui est soumise en contraction par un élément piézo-électrique (16) déclenché par des impulsions de dosage générées en fonction d'une quantité distribuée prévue par unité de temps.

2. Procédé selon la revendication 1, **caractérisé en ce que** le courant gazeux matriciel qui contient les microgouttelettes est chauffé.

3. Procédé selon la revendication 1 ou 2, **caractérisé en ce que** l'expansion volumétrique provoquée par la vaporisation et le cas échéant par le chauffage est prise en compte lors de la commande du débit quantitatif du courant gazeux matriciel.

4. Procédé selon l'une des revendications 1 à 3, **caractérisé en ce que** l'on utilise en guise de gaz matriciel un gaz de haute pureté, en particulier de l'air synthétique ou de l'azote.

5. Procédé selon l'une des revendications 1 à 4, **caractérisé en ce que** l'on introduit des microgouttelettes de taille unitaire avec un diamètre dans la plage d'environ 30 à 50 µm.

6. Procédé selon l'une des revendications 1 à 5, **caractérisé en ce que** les microgouttelettes sont emportées depuis les buses (4) par un courant partiel du gaz matriciel qui s'écoule en douceur autour des buses (4).

7. Procédé selon l'une des revendications 1 à 6, **caractérisé en ce que** l'on introduit une ou plusieurs composantes au goutte-à-goutte sous la forme d'une solution contenant ces composantes en concentration prédéterminée.

8. Appareil pour préparer un mélange gazeux contenant au moins une composante gazeuse de traçage en concentration prédéterminée, en particulier un gaz de calibrage, comprenant une source de gaz matriciel (1) suivie par un régulateur de courant massique, et comprenant au moins une source (2) pour une composante, ainsi qu'un dispositif de dosage (3) pour ladite au moins une composante de traçage, dans lequel le dispositif de dosage (3) est un dispositif de dosage à microgouttelettes comprenant au moins une buse (4) qui distribue des microgouttelettes individuelles en succession, ladite buse (4) débouchant dans un tube de vaporisation (6) traversé par le gaz matriciel et comprenant un élément piézo-électrique (16) dans le but de générer les microgouttelettes en tant que moyen pour solliciter la buse (4) en contraction, ledit élément piézo-électrique (16) étant couplé à une commande (17) qui déclenche l'élément piézo-électrique (16) avec des impulsions de dosage en correspondance de la quantité distribuée prévue par unité de temps.

9. Appareil selon la revendication 8, **caractérisé en ce que** le tube de vaporisation (6) est chauffable.

10. Appareil selon la revendication 8 ou 9, **caractérisé en ce que** pour le dispositif de dosage (3) à microgouttelettes, il est prévu une commande numérique (S) pour la longueur et la succession des impulsions de dosage.

11. Appareil selon l'une des revendications 8 à 10, **caractérisé en ce que** les buses (4) sont susceptibles d'être léchées par un courant partiel du gaz matriciel en douceur dirigé vers le tube de vaporisation (6).

12. Procédé pour calibrer un système d'analyse gazeuse, en particulier un chromatographe à gaz (20) par traversée et analyse d'un gaz matriciel sous forme gazeuse qui contient au moins une composante de calibrage, dans lequel un courant gazeux matriciel est introduit dans le système avec un débit quantitatif prédéterminé, on introduit dans le courant gazeux matriciel avant sa traversée à travers le système au moins une composante de calibrage en quantité prédéterminée par unité de volume du gaz matriciel, sous la forme de microgouttelettes essentiellement de même taille, et on les fait vaporiser dans le gaz matriciel pour réaliser un gaz de calibrage, les microgouttelettes sont distribuées par au moins une buse (4) qui est sollicité en contraction par un élément piézo-électrique (16) déclenché par des impulsions de dosage générées en fonction de la quantité distribuée prévue par unité de temps.

13. Procédé selon la revendication 12, **caractérisé en ce que** le courant de gaz matriciel est chauffé avec les microgouttelettes.

14. Procédé selon la revendication 12 ou 13, **caractérisé en ce que** l'expansion volumétrique provoquée par la vaporisation et le cas échéant par le chauffage est prise en compte lors de la commande du débit quantitatif du courant de gaz matriciel.

15. Procédé selon l'une des revendications 12 à 14, **caractérisé en ce que** l'on utilise en guise de gaz matriciel un gaz de haute pureté, en particulier de l'air synthétique ou de l'azote.

16. Procédé selon l'une des revendications 12 à 15, **caractérisé en ce que** l'on introduit des microgouttelettes de taille unitaire avec un diamètre dans la plage d'environ 30 à 50 µm.

17. Procédé selon l'une des revendications 12 à 16, **caractérisé en ce que** les microgouttelettes sont emportées depuis les buses (4) par un courant partiel du gaz matriciel qui s'écoule en douceur autour des buses (4).

18. Procédé selon l'une des revendications 12 à 17, **caractérisé en ce que** l'on introduit une ou plusieurs composantes de calibrage au goutte-à-goutte sous la forme d'une solution contenant ces composantes de calibrage en concentration prédéterminée.

19. Procédé selon l'une des revendications 1 à 7 ou 12 à 18, **caractérisé en ce que** la concentration de la ou des composantes de traçage est modifiée par modification de la fréquence des impulsions de déclenchement.
